# EUROPÄISCHE PATENTSCHRIFT

(11) **EP 0 449 015 B1**
(45) Veröffentlichungstag und Bekanntmachung des Hinweises auf die Patenterteilung: **01.07.1998**
(21) Anmeldenummer: 91103627.5
(22) Anmeldetag: 09.03.1991
(51) Int. Cl.: C07D 239/26, C09K 19/30, C07D 213/26, C09K 19/12, C09K 19/14, C09K 19/34, C07C 25/18, C07C 43/225, C09K 19/32, C07C 22/08

(54) **Difluormethylverbindungen und flüssigkristallines Medium**
Difluoromethyl compounds and liquid criystal medium
Composés difluorométhylés et milieu cristal liquide

(30) Priorität: 24.03.1990 DE 4009564
(43) Veröffentlichungstag der Anmeldung: 02.10.1991
(73) Patentinhaber: MERCK PATENT GmbH, 64271 Darmstadt (DE)
(72) Erfinder: Bartmann, Ekkehard, Dr., W-6106 Erzhausen (DE); Dorsch, Dieter, Dr., W-6100 Darmstadt (DE); Poetsch, Eike, Dr., W-6109 Mühltal 6 (DE); Hittich, Reinhard, Dr., W-6101 Modautal 1 (DE); Rieger, Bernhard, Dr., WACORE-Tamagawagakuen, Yokohama-shi, Kanagawa-Pref. 227 (JP)

(56) Entgegenhaltungen:
- EP-A- 255 236
- WO-A-87/06602
- WO-A-89/02884
- WO-A-90/01056
- WO-A-91/03450
- DE-A- 3 904 817

## Beschreibung

Die Erfindung betrifft neue Difluormethylverbindungen der Formel I, 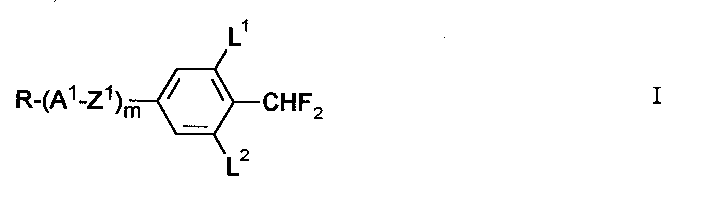 worin
- R: einen unsubstituierten oder einen mindestens einfach durch Halogen substituierten Alkyl-oder Alkenylrest mit 1 bis 15 C-Atomen, wobei in diesen Resten auch eine oder mehrere CH₂-Gruppen jeweils unabhängig voneinander durch -O-, -S-, 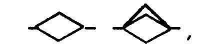 -CO-, -CO-O-, -O-CO-oder -O-CO-O- so ersetzt sein können, daß O-Atome nicht direkt miteinander verknüpft sind,
- A¹ und A²: jeweils unabhängig voneinander einen
(a) trans-1,4-Cyclohexylenrest, worin auch eine oder mehrere nicht benachbarte CH₂-Gruppen durch -O- und/oder -S- ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) und (b) mit CN oder 1-2 Fluor substituiert sein können,
- L¹ und L²: jeweils unabhängig voneinander H oder F,
- Z¹: -CO-O-, -O-CO-, -CH₂O-, -OCH₂-, -CH₂CH₂-, -CH=CH-, -C≡C- oder eine Einfachbindung und
- m: 0, 1, 2 oder 3 bedeutet,
mit der Maßgabe für den Vertragsstaat DE, daß L¹ und/oder L² F bedeuten.

Die Erfindung betrifft weiterhin die Verwendung dieser Verbindungen als Komponenten flüssigkristalliner Medien sowie Flüssigkristall- und elektrooptische Anzeige, die die erfindungsgemäßen flüssigkristallinen Medien enthalten.

Die Verbindungen der Formel I können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Medien geeignet sind und insbesondere gleichzeitig eine vergleichsweise geringe Viskosität besitzen sowie eine relativ hohe dielektrische Anisotropie.

Es wurde nun gefunden, daß Verbindungen der Formel I als Komponenten flüssigkristalliner Medien vorzüglich geeignet sind. Insbesondere verfügen sie über vergleichsweise niedere Viskositäten. Mit ihrer Hilfe lassen sich stabile flüssigkristalline Medien mit breitem Mesophasenbereich und vorteilhaften Werten für die optische und dielektrische Anisotropie erhalten.

Flüssigkristalle der folgenden Formeln: 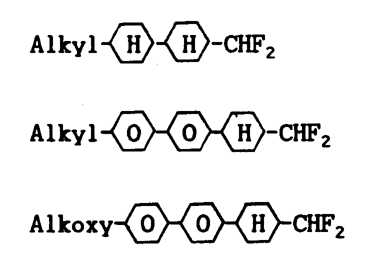 sind bereits aus DE 39 09 802 bekannt.

Im Hinblick auf die verschiedensten Einsatzbereiche derartiger Verbindungen war es jedoch wünschenswert, weitere Verbindungen zur Verfügung zu haben, die auf die jeweiligen Anwendungen genau maßgeschneiderte Eigenschaften aufweisen.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/ oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Verbindungen der Formel I sowie die Verwendung dieser Verbindungen als Komponenten flüssigkristalliner Medien. Gegenstand der Erfindung sind ferner flüssigkristalline Medien mit einem Gehalt an mindestens einer Verbindung der Formel I sowie Flüssigkristallanzeigeelemente, insbesondere elektroopische Anzeigeelemente, die derartige Medien enthalten.

Der Einfachheit halber bedeuten im folgenden Cyc einen 1,4-Cyclohexylenrest, Che einen 1,4-Cyclohexenylenrest, Dio einen 1,3-Dioxan-2,5-diylrest, Dit einen 1,3-Dithian-2,5-diylrest, Phe einen 1,4-Phenylenrest, Pyd einen Pyridin-2,5-diylrest, Pyr einen Pyrimidin-2,5-diylrest und Bi einen Bicyclo(2,2,2)-octylenrest.

Die Verbindungen der Formel I umfassen dementsprechend die bevorzugten Verbindungen der Teilformeln Ia, Ib und Ic

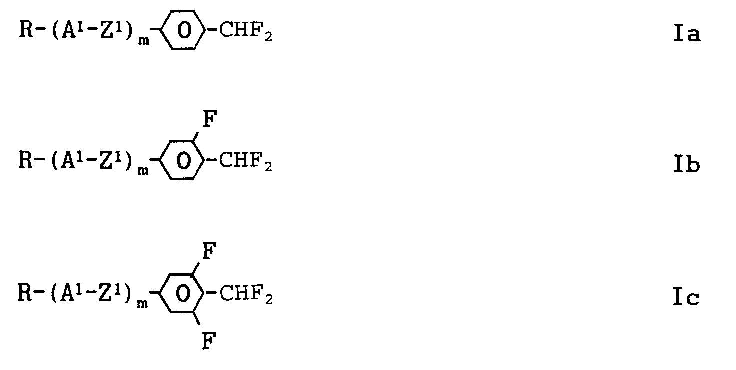

Darunter sind besonders diejenigen der Teilformel Ic bevorzugt.

R bedeutet vorzugsweise Alkyl, ferner Alkoxy. A¹ bedeutet bevorzugt Phe, Cyc, Che, Pyr oder Dio. Bevorzugt enthalten die Verbindungen der Formel I nicht mehr als einen der Reste Bi, Pyd, Pyr, Dio oder Dit.

Bevorzugt sind auch Verbindungen der Formel I sowie aller Teilformeln, in denen A¹ ein- oder zweifach durch F oder einfach durch CN substituiertes 1,4-Phenylen bedeutet. Insbesondere sind dies 2-Fluor-1,4-phenylen, 3-Fluor-1,4-phenylen und 2,3-Difluor-1,4-phenylen sowie 2-Cyan-1,4-phenylen und 3-Cyan-1,4-phenylen.

Z¹ bedeutet bevorzugt eine Einfachbindung, -CO-O-, -O-CO- und -CH₂CH₂-, in zweiter Linie bevorzugt -CH₂O- und -OCH₂-. Vorzugsweise ist nur eine der im Molekül vorhandenen Gruppen Z¹ von der Einfachbindung verschieden.

Falls R einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R einen Alkylrest bedeutet, in dem eine CH₂-Gruppe durch -CH=CH- ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R einen Alkylrest bedeutet, in dem eine CH₂-Gruppe durch -O- und eine durch -CO- ersetzt ist, so sind diese bevorzugt benachbart. Somit beeinhalten diese eine Acyloxygruppe -CO-O- oder eine Oxycarbonylgruppe -O-CO-. Vorzugsweise sind diese geradkettig und haben 2 bis 6 C-Atome.

Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R einen Alkylrest bedeutet, in dem eine CH₂-Gruppe durch unsubstituiertes oder substituiertes -CH=CH- und eine benachbarte CH₂-Gruppe durch CO oder CO-O oder O-CO-ersetzt ist, so kann dieser geradkettig oder verzweigt sein. Vorzugsweise ist er geradkettig und hat 4 bis 13 C-Atome. Er bedeutet demnach besonders Acryloyloxymethyl, 2-Acryloyloxyethyl, 3-Acryloyloxypropyl, 4-Acryloyloxy butyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3- Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Verbindungen der Formel I, die über für Polymerisationsreaktionen geeignete Flügelgruppen R verfügen, eignen sich zur Darstellung flüssigkristalliner Polymerer.

Verbindungen der Formel I mit verzweigten Flügelgruppen R können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verbindungen der Formel I mit S_{A}-Phasen eignen sich beispielsweise für thermisch adressierte Displays.

Verzweigte Gruppen dieser Art enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R einen Alkylrest darstellt, in dem zwei oder mehr CH₂-Gruppen durch -O- und/oder -CO-O- ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxy-ethyl, 3,3-Bis-carboxy-propyl, 4,4-Bis-carboxy-butyl, 5,5-Bis-carboxy-pentyl, 6,6-Bis-carboxy-hexyl, 7,7-Bis-carboxy-heptyl, 8,8-Bis-carboxy-octyl, 9,9-Bis-carboxy-nonyl, 10,10-Bis-carboxy-decyl, Bis-(methoxycarbonyl)-methyl, 2,2-Bis-(methoxycarbonyl)-ethyl, 3,3-Bis-(methoxycarbonyl)-propyl, 4,4-Bis-(methoxycarbonyl)-butyl, 5,5-Bis-(methoxycarbonyl)-pentyl, 6,6-Bis-(methoxycarbonyl)-hexyl, 7,7-Bis-(methoxycarbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl, 4,4-Bis-(ethoxycarbonyl)-butyl, 5,5-Bis-(ethoxycarbonyl)-hexyl.

Verbindungen der Formel I, die über für Polykondensationen geeignete Flügelgruppen R verfügen, eignen sich zur Darstellung flüssigkristalliner Polykondensate.

Formel I umfaßt sowohl die Racemate dieser Verbindungen als auch die optischen Antipoden sowie deren Gemische.

-(A¹-Z¹)ₘ- hat vorzugsweise eine der folgenden Bedeutungen:

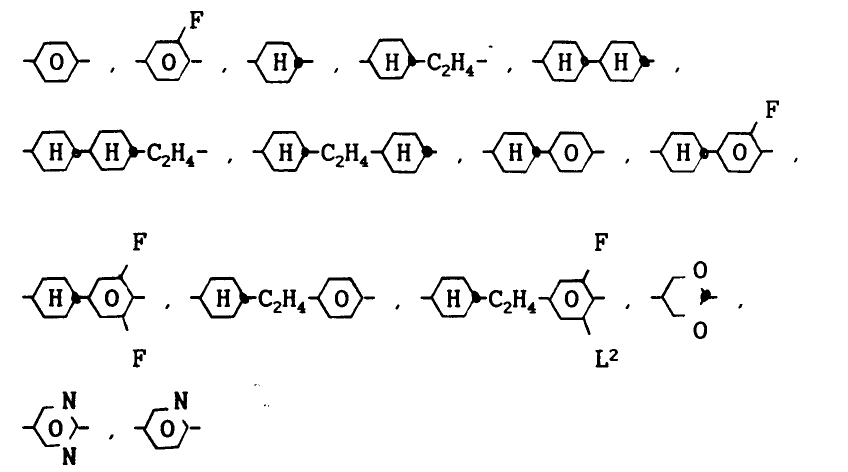

Unter diesen Verbindungen der Formel I sowie den Unterformeln sind diejenigen bevorzugt, in denen mindestens einer der darin enthaltenden Reste eine der angegebenen bevorzugten Bedeutungen hat.

In den Verbindungen der Formel I sind diejenigen Stereoisomeren bevorzugt, in denen die Ringe Cyc und Piperidin trans-1,4-disubstituiert sind. Diejenigen der vorstehend genannten Formeln, die eine oder mehrere Gruppen Pyd, Pyr und/oder Dio enthalten, umschließen jeweils die beiden 2,5-Stellungsisomeren.

Die 1,4-Cyclohexenylen-Gruppe hat vorzugsweise folgende Strukturen:

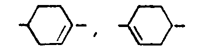

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart Bd IX, S. 867 ff.) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Verbindungen der Formel I können z.B. hergestellt werden, indem man entsprechende Aldehyde (die ihrerseits aus entsprechenden, bekannten Nitrilen nach Routinemethoden zugänglich sind) mit SF₄ umsetzt.

Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Verbindungen der Formel I, die ansonsten der Formel I entsprechen, aber an Stelle von 1,4-Phenylenresten 1,4-Cyclo-hexenylenreste besitzen, können zum Beispiel mit DDQ (Dichlordicyanobenzochinon) in einem geeigneten Lösungsmittel oxidiert werden.

Ester der Formel I können auch durch Veresterung entsprechender Carbonsäuren (oder ihren reaktionsfähigen Derivate) mit Alkoholen bzw. Phenolen (oder ihren reaktionsfähigen Derivaten) oder nach der DCC-Methode (DCC = Dicyclohexylcarbodiimid) erhalten werden.

Die entsprechenden Carbonsäuren und Alkohole bzw. Phenole sind bekannt oder können in Analogie zu bekannten Verfahren hergestellt werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Natrium oder Kalium, in Betracht.

Die Veresterung wird vorteilhaft in Gegenwart eines inerten Lösungsmittels durchgeführt. Gut geeignet sind insbesondere Ether wie z.B. Diethylether, Di-n-butylether, THF, Dioxan oder Anisol, Ketone wie z.B. Aceton, Butanon oder Cyclohexanon, Amide wie z.B. DMF oder Phosphorsäurehexamethyltriamid, Kohlenwasserstoffe wie z.B. Benzol, Toluol oder Xylol, Halogenkohlenwasserstoffe wie z.B. Tetrachlorkohlenstoff, Dichlormethan oder Tetrachlorethylen und Sulfoxide wie z.B. Dimethylsulfoxid oder Sulfolan.

Ether der Formel I sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, NaNH₂, NaOH, KOH, Na₂CO₃ oder K₂CO₃ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie z.B. Aceton, 1,2-Dimethoxyethan, DMF oder Dimethylsulfoxid oder auch mit einem Überschuß an wäßriger oder wäßrig-alkoholischer NaOH oder KOH bei Temperaturen zwischen etwa 20° und 100°.

Dioxanderivate bzw. Dithianderivate der Formel I werden zweckmäßig durch Reaktion eines entsprechenden Aldehyds (oder eines seiner reaktionsfähigen Derivate) mit einem entsprechenden 1,3-Diol (oder einem seiner reaktionsfähigen Derivate) bzw. einem entsprechenden 1,3-Dithiol hergestellt, vorzugsweise in Gegenwart eines inerten Lösungsmittels wie z.B. Benzol oder Toluol und/oder eines Katalysators, z.B. einer starken Säure wie Schwefelsäure, Benzol- oder p-Toluolsulfonsäure, bei Temperaturen zwischen etwa 20° und etwa 150°, vorzugsweise zwischen 80° und 120°. Als reaktionsfähige Derivate der Ausgangsstoffe eignen sich in erster Linie Acetale.

Die genannten Aldehyde und 1,3-Diole bzw. 1,3-Dithiole sowie ihre reaktionsfähigen Derivate sindzum Teil bekannt, zum Teil können sie ohne Schwierigkeiten nach Standardverfahren der Organischen Chemie aus literaturbekannten Verbindungen hergestellt werden. Beispielsweise sind die Aldehyde durch Oxydation entsprechender Alkohole oder durch Reduktion von Nitrilen oder entsprechenden Carbonsäuren oder ihrer Derivate, die Diole durch Reduktion entsprechender Diester und die Dithiole durch Umsetzung entsprechender Dihalogenide mit NaSH erhältlich.

Die erfindungsgemäßen Verbindungen mit L¹ = F können z.B. hergestellt werden, indem man eine Verbindung der Formel II, 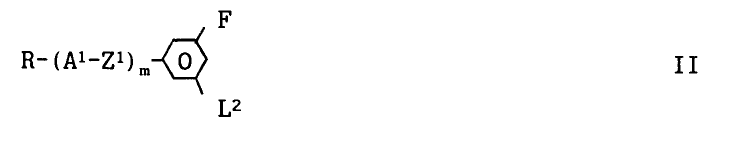 worin R, A¹, Z¹, L² und m die angegebene Bedeutung haben, gemäß folgenden Reaktionsschemata umsetzt:

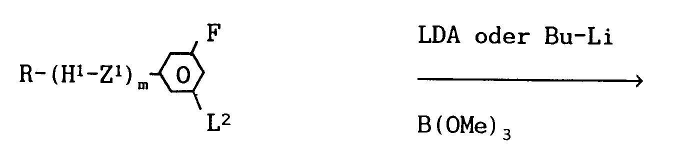

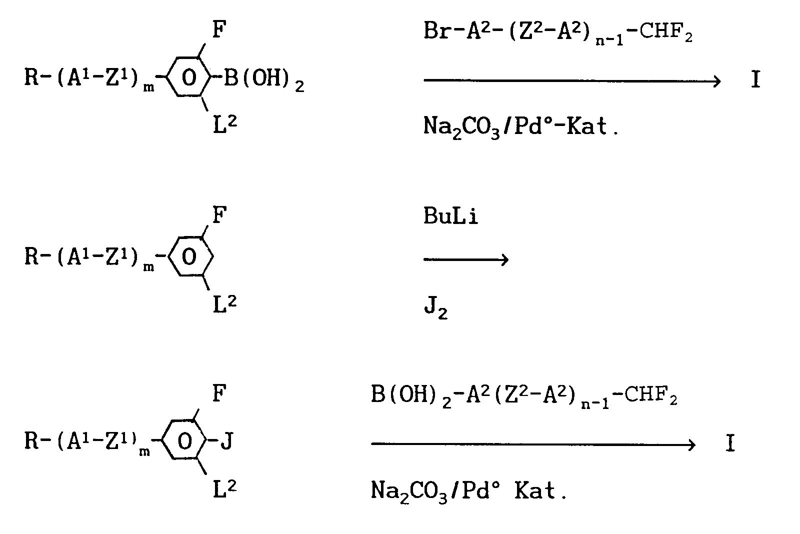

Weitere Synthesemethoden sind für den Fachmann augenscheinlich. Beispielsweise können in 5-Position entsprechend substituierte 1,3-Difluorbenzol-Verbindungen oder monofluorierte Analoga (L² = H) gemäß obigem Schema in die 2-substituierten 1,3-Difluorverbindungen oder monofluorierte Analoga (L² = H) überführt werden und der Rest R-(A¹-Z¹)ₘ-anschließend durch in der Flüssigkristallchemie gebräuchliche Reaktionen (z.B. Veresterung, Veretherung oder Kopplungen z.B. gemäß der Artikel E. Poetsch, Kontakte (Darmstadt) 1988 (2), S. 15) eingeführt werden oder umgekehrt.

Die Verbindungen der Formel II können beispielsweise nach folgenden Syntheseschemata hergestellt werden:

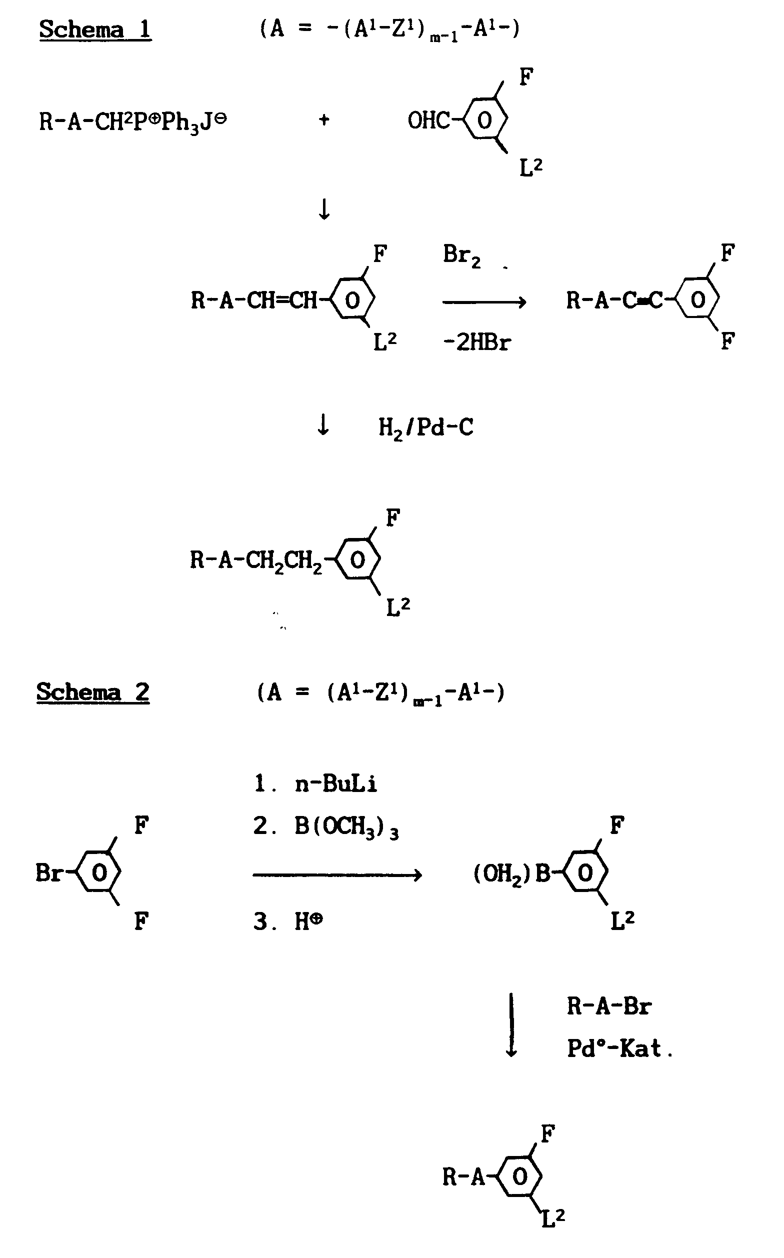

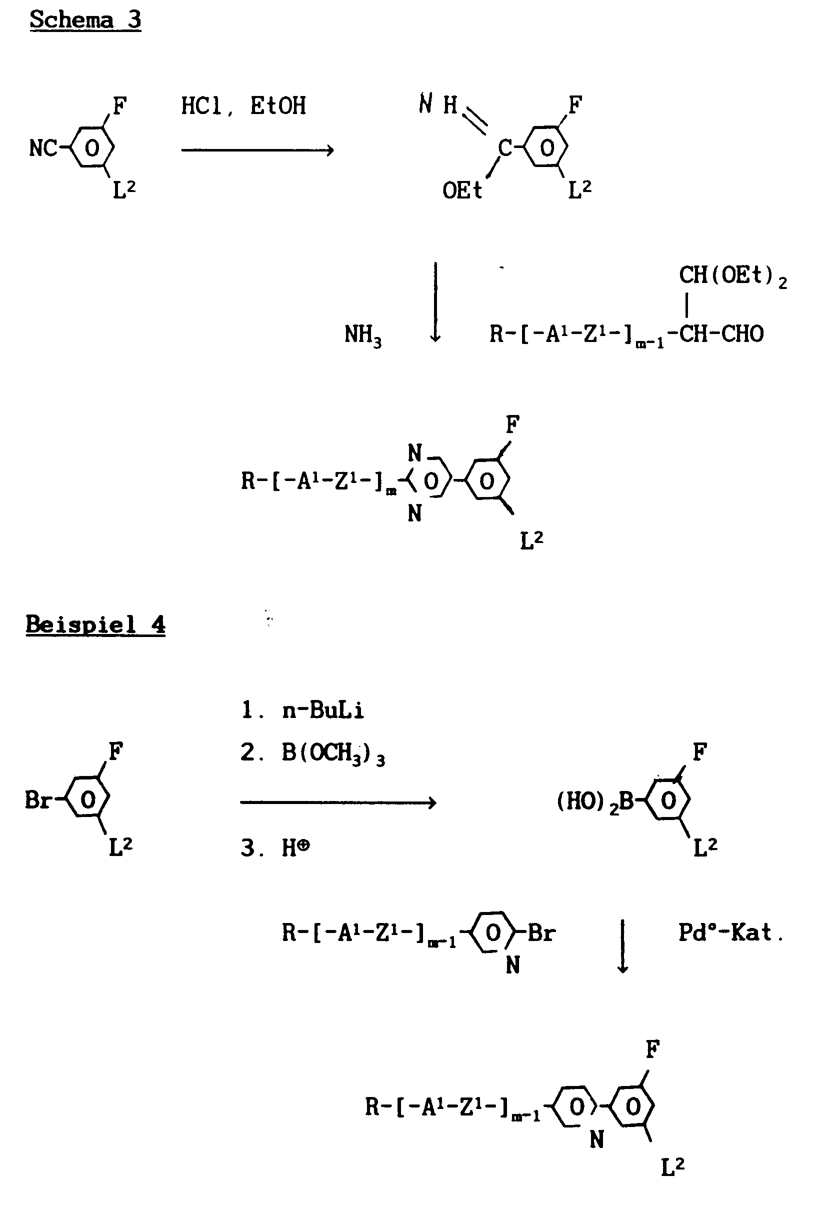

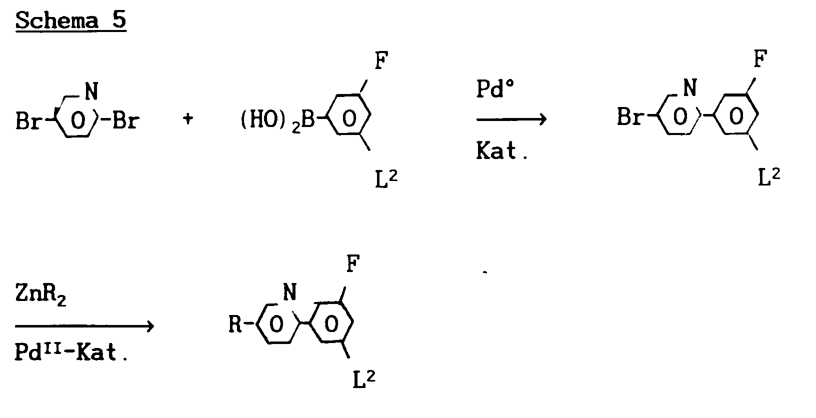

Die Ausgangsmaterialien sind entweder bekannt oder können in Analogie zu bekannten Verbindungen hergestellt werden.

Ester der Formel I können auch durch Veresterung entsprechender Carbonsäuren (oder ihrer reaktionsfähigen Derivate) mit Alkoholen bzw. Phenolen (oder ihren reaktionsfähigen Derivaten) oder nach der DCC-Methode (DCC = Dicyclohexylcarbodiimid) erhalten werden.

Die entsprechenden Carbonsäuren und Alkohole bzw. Phenole sind bekannt oder können in Analogie zu bekannten Verfahren oder auch durch Umsetzung von metalliertem II mit geeigneten Elektrophilen (B(OH)₃/H₂O₂ bzw. CO₂) hergestellt werden.

Die Synthese einiger besonders bevorzugter Verbindungen ist im folgenden näher angegeben (Y = H oder F):

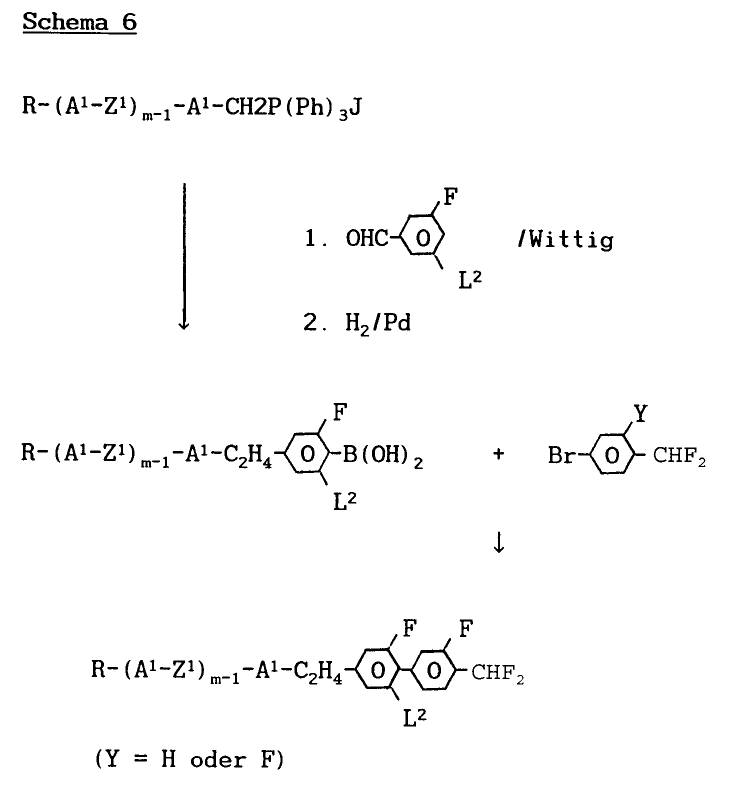

In einem weiteren Verfahren zur Herstellung der Verbindungen der Formel I setzt man ein Arylhalogenid mit einem Olefin um in Gegenwart eines tertiären Amins und eines Palladiumkatalysators (vgl. R.F. Heck, Acc. Chem. Res. 12 (1979) 146). Geeignete Arylhalogenide sind beispielsweise Chloride, Bromide und Iodide, insbesondere Bromide und Iodide. Die für das Gelingen der Kupplungsreaktion erforderlichen tertiären Amine, wie z.B. Triethylamin, eignen sich auch als Lösungsmittel. Als Palladiumkatalysatoren sind beispielsweise dessen Salze, insbesondere Pd(II)-acetat, mit organischen Phosphor(III)-Verbindungen wie z.B. Triarylphosphanen geeignet. Man kann dabei in Gegenwart oder Abwesenheit eines inerten Lösungsmittels bei Temperaturen zwischen etwa 0° und 150°, vorzugsweise zwischen 20° und 100°, arbeiten; als Lösungsmittel kommen z.B. Nitrile wie Acetonitril oder Kohlenwasserstoffe wie Benzol oder Toluol in Betracht. Die als Ausgangsstoffe eingesetzten Arylhalogenide und Olefine sind vielfach im Handel erhältlich oder können nach literaturbekannten Verfahren hergestellt werden, beispielsweise durch Halogenierung entsprechender Stammverbindungen bzw. durch Eliminierungsreaktionen an entsprechenden Alkoholen oder Halogeniden.

Auf diese Weise sind beispielsweise Stilbenderivate herstellbar. Die Stilbene können weiterhin hergestellt werden durch Umsetzung eines 4-substituierten Benzaldehyds mit einem entsprechenden Phosphorylid nach Wittig. Man kann aber auch Tolane der Formel I herstellen, indem man anstelle des Olefins monosubstituiertes Acetylen einsetzt (Synthesis 627 (1980) oder Tetrahedron Lett. 27, 1171 (1986)).

Weiterhin können zur Kopplung von Aromaten Arylhalogenide mit Arylzinkverbindungen umgesetzt werden. Bevorzugt werden diese Reaktionen unter Zusatz eines Katalysators wie z.B. eines Palladium(O)komplexes in inerten Lösungsmitteln wie Kohlenwasserstoffen bei hohen Temperaturen, z.B. in siedendem Xylol, unter Schutzgas durchgeführt.

Kopplungen von Alkinyl-Verbindungen mit Arylhalogeniden können analog dem von A.O. King, E. Negishi, F.J. Villani und A. Silveira in J.Org.Chem. 43, 358 (1978) beschriebenen Verfahren durchgeführt werden.

Tolane der Formel I können auch über die Fritsch-Buttenberg-Wiechell-Umlagerung (Ann. 279, 319, 332, 1984) hergestellt werden, bei der 1,1-Diaryl-2-halogenethylene umgelagert werden zu Diarylacetylenen in Gegenwart starker Basen.

Tolane der Formel I können auch hergestellt werden, indem man die entsprechenden Stilbene bromiert und anschließend einer Dehydrohalogenierung unterwirft. Dabei kann man an sich bekannte, hier nicht näher erwähnte Varianten dieser Umsetzung anwenden.

Ether der Formel I sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, NaNH₂, NaOH, KOH, Na₂CO₃ oder K₂CO₃ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel, wie z.B. Aceton, 1,2-Dimethoxyethan, DMF oder Dimethylsulfoxid oder auch mit einem Überschuß an wäßriger oder wäßrig-alkoholischer NaOH oder KOH bei Temperaturen zwischen etwa 20° und 100°.

Ausgehend von dem käuflichen 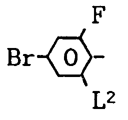 lassen sich die Verbindungen nach an und für sich bekannten Methoden darstellen. Außer den im nachstehenden Schema aufgeführten Verknüpfungsmöglichkeiten ergeben sich auch analoge Syntheseverfahren über Friedel-Crafts-Acylierung oder Wittig-Reaktion mit nachfolgender Reduktion bzw. Hydrierung für den Aufbau der CH₂-CH₂ verknüpften Substanzen.

Die 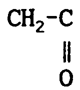 enthaltenden Verbindungen lassen sich über eine Chlorierung mittels PCl₅ zu 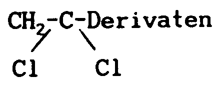 und nachfolgende HCl-Eliminierung mittels Base in die Acetylene umwandeln. Die Transformation der -CH=CH- verbrückten Substanzen, die über Heck-Kopplung oder Wittig-Reaktion herstellbar sind, gelingt nach herkömmlicher Weise z.B. mittels Bromierung und doppelter HBr-Abspaltung.

Die Einführung der Carboxylat-Gruppe gelingt in Analogie zur Einführung der CH₂CH₂-Gruppe (vergl. Verbindung 7, nachstehendes Formelschema) mittels CO₂. Die intermediär gebildeten Säuren lassen sich mit den entsprechenden Phenolen verestern, die aus 1 gemäß Syntheseschritt ba darstellbar sind.

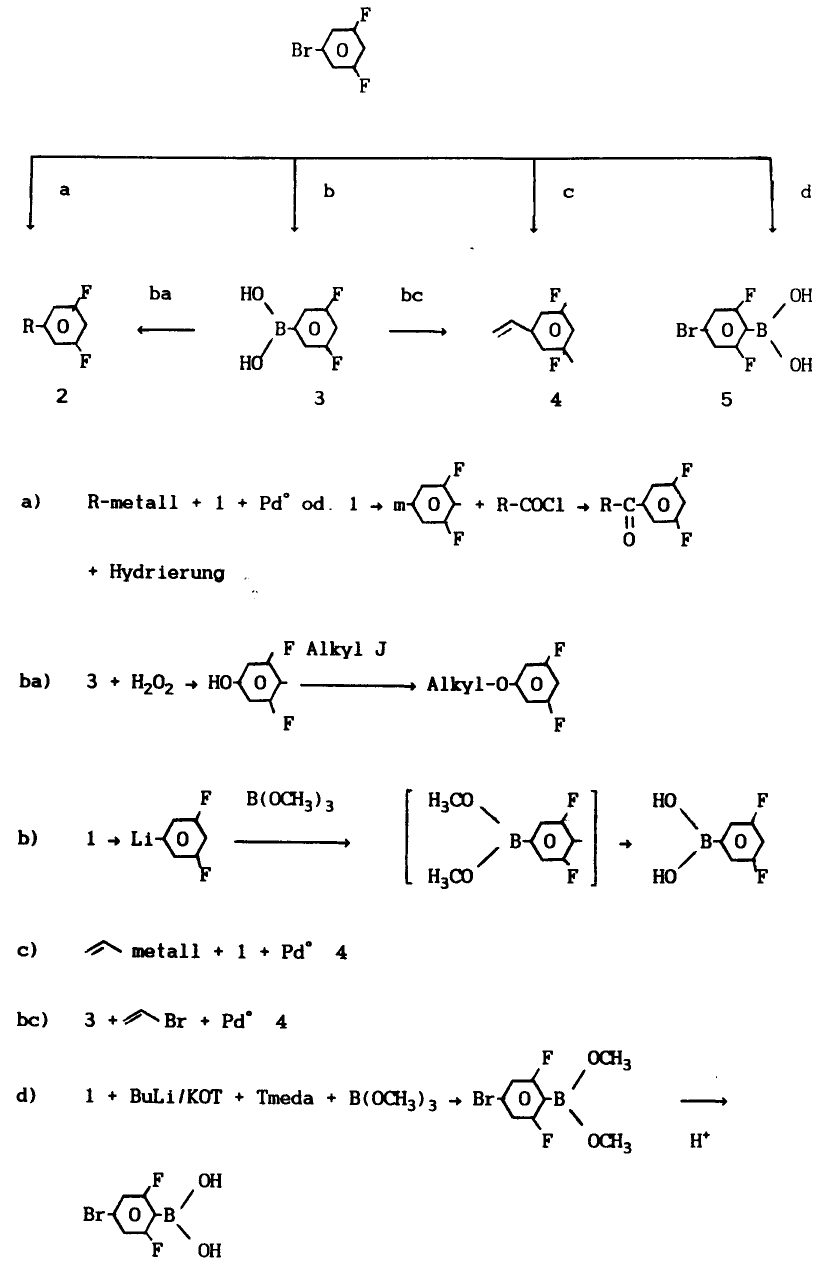

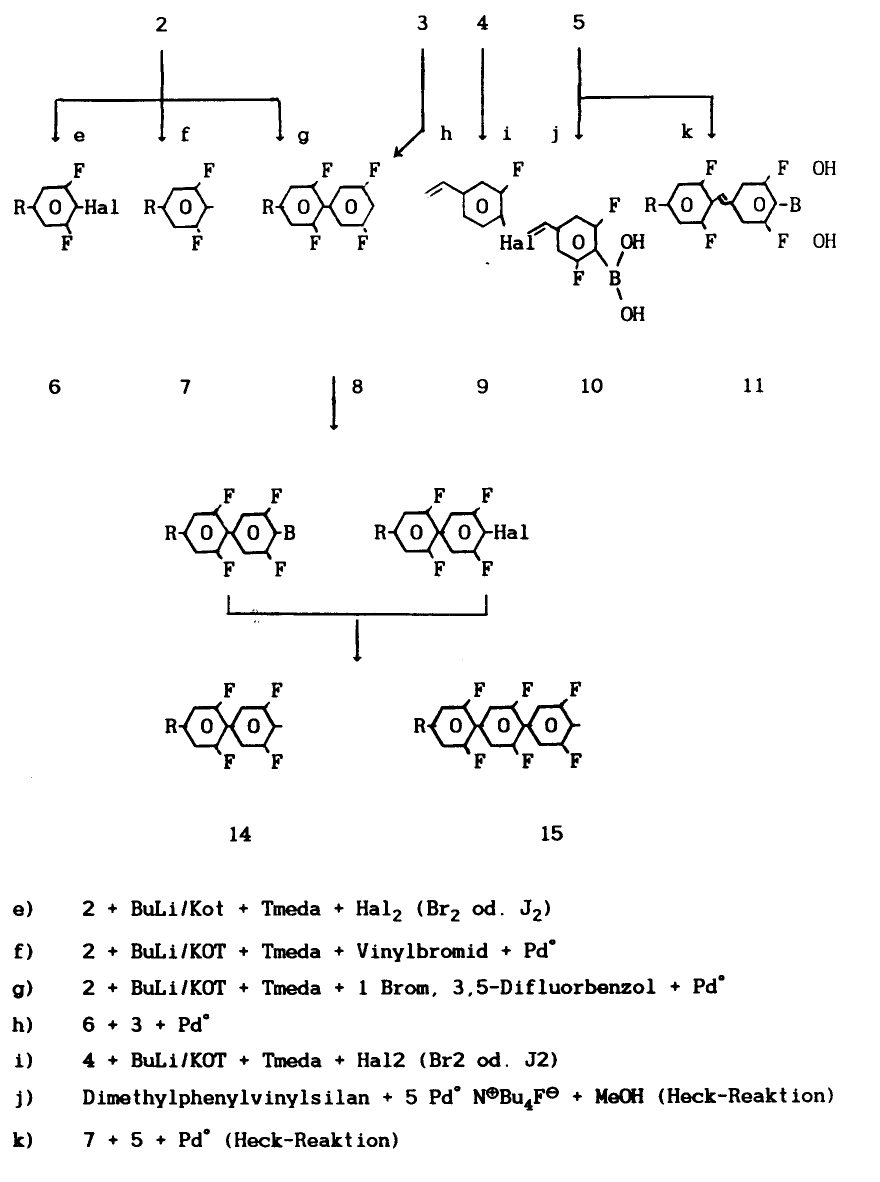

Die Verbindungen der Formel I, worin L¹ und L² F bedeuten und der mit 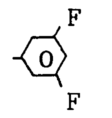 verknüpfte Rest A¹ 1,4-Cyclohexylen bedeutet, können nach folgendem Schema hergestellt werden:

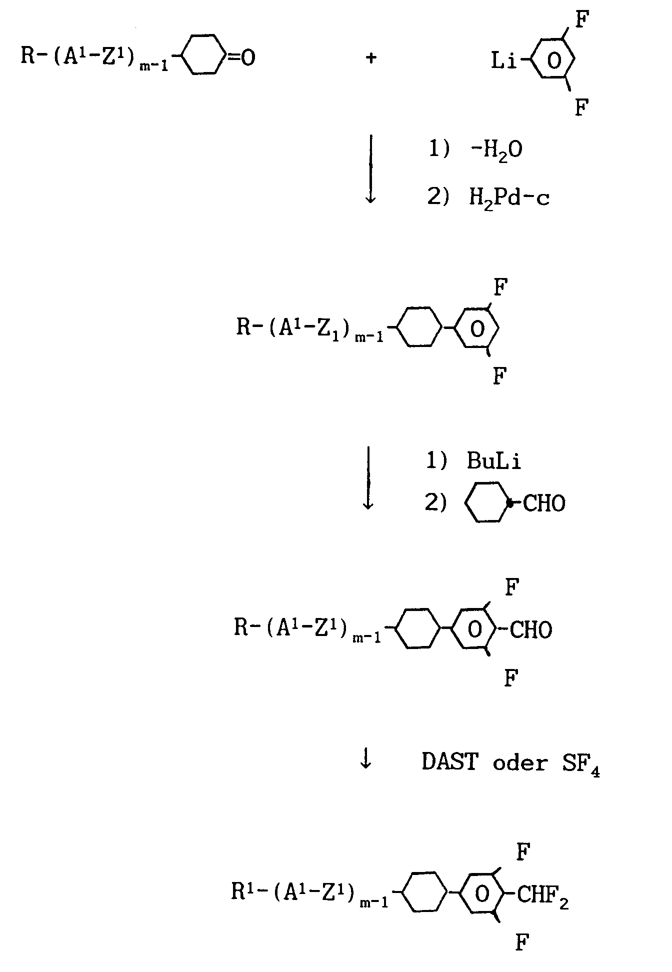

Die Verbindung 8 läßt sich gemäß d) zu 12 und analog e) zu 13 umsetzen. 12 wird mittels Vinylbromid gemäß bc) und 13 mittels Divinylzink gemäß c) zu 14 umgesetzt. 15 läßt sich aus 12 und 1 mittels Kreuzkopplung gemäß h) erhalten oder aus 13 und 3 durch Kreuzkopplung.

Die CH=CH-Gruppierung ist in die CH₂-CH₂ oder -C≡C-Gruppierung überführbar.

Die Einführung der Endgruppierung 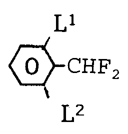 gelingt entweder durch Verwendung eines Kopplungsschrittes mit 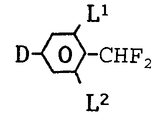 (D = Hal, m, 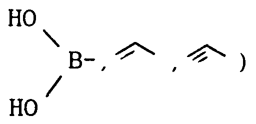 oder durch Einführung von CHF₂ in Verbindungen mit der Endgruppierung

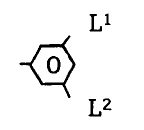

Die Herstellung von ebenfalls die CO₂ und/oder C≡C Zwischenglieder enthaltenden Verbindungen läßt sich sinngemäß nach analogen Formelschemata durchführen.

Einige besonders bevorzugt, kleinere Gruppen von Verbindungen der Formel I sind im folgenden angegeben:

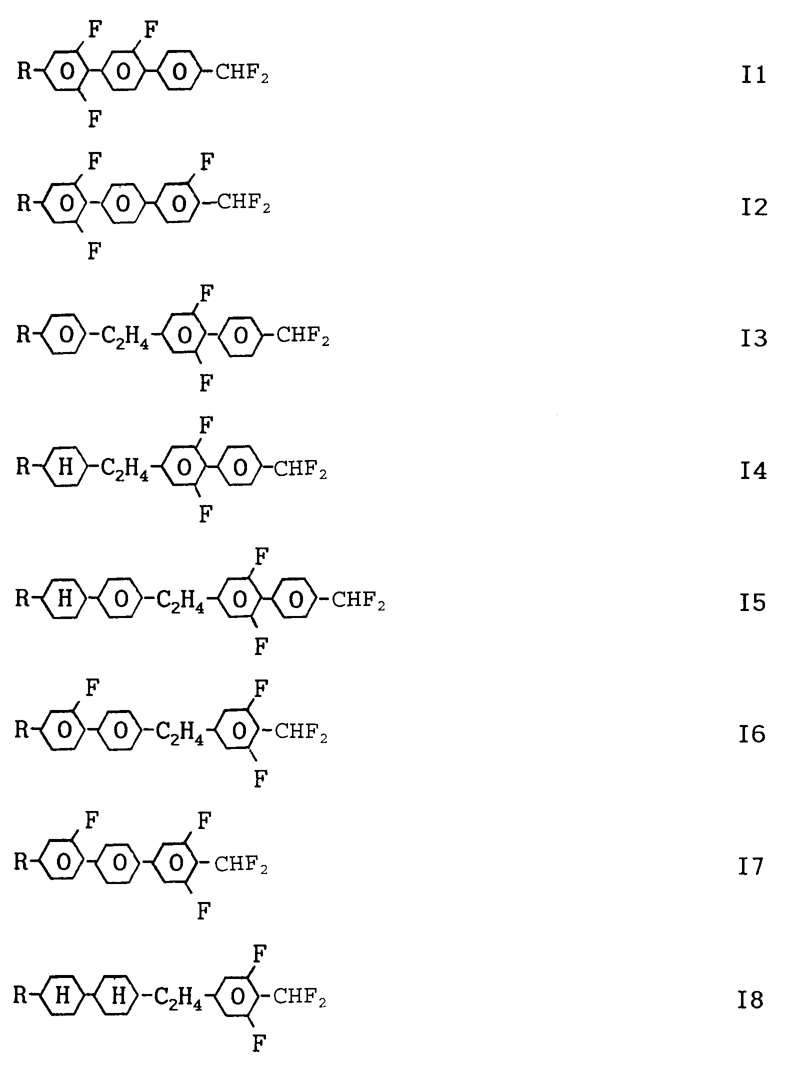

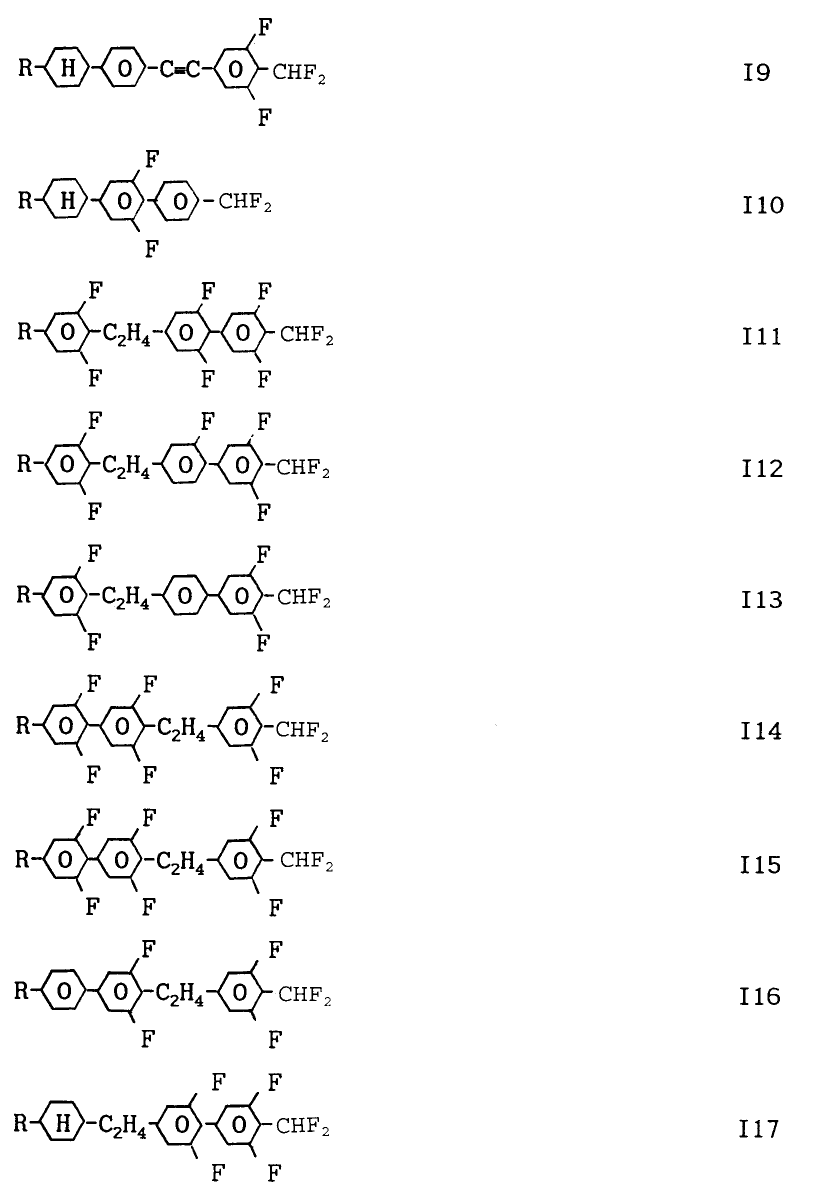

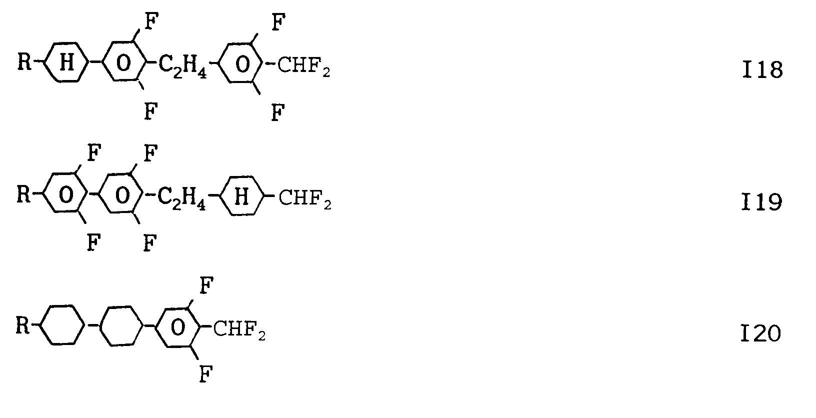

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane Cyclohexylcyclohexene, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4, und 5 charakterisieren:

R'-L-E-R'' 1

R'-L-COO-E-R'' 2

R'-L-OOC-E-R'' 3

R'-L-CH₂CH₂-E-R'' 4

R'-L-C≡C-E-R'' 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R'' bedeuten in den Verbindungen der Teilformeln 1a, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Verbindungen sind R' und R'' voneinander verschieden, wobei einer dieser Reste meist Alkyl oder Alkenyl ist. In den Verbindungen der Teilformeln 1b, 2b, 3b, 4b und 5b bedeutet R'' -CN, -CF₃, F, Cl oder -NCS; R hat dabei die bei den Verbindungen der Teilformeln 1a bis 5a angegebene Bedeutung und ist vorzugsweise Alkyl oder Alkenyl. Aber auch andere Varianten der vorgesehenen Substituenten in den Verbindungen der Formeln 1, 2, 3, 4 und 5 sind gebräuchlich. Viele solcher Substanzen oder auch Gemische davon sind im Handel erhältlich. Alle diese Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:
Gruppe 1: 20 bis 90 %, insbesondere 30 bis 90%,
Gruppe 2: 10 bis 80 %, insbesondere 10 bis 50 %,
   wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. Vor- und nachstehend bedeuten Potenzangaben Gewichtsprozent. Alle Temperaturen sind in Grad Celsius angegeben. Fp. bedeutet Schmelzpunkt Kp = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. n bedeutet optische Anisotropie (589 nm, 20 °C) und die Viskosität (mm²/sec) wurde bei 20 °C bestimmt.

"Übliche Aufarbeitung" bedeutet: man gibt gegebenenfalls Wasser hinzu, extrahiert mit Methylenchlorid, Diethylether oder Toluol, trennt ab, trocknet die organische Phase, dampft ein und reinigt dar Produkt durch Destillation unter reduziertem Druck oder Kristallisation und/oder Chromatographie. Folgende Abkürzungen werden verwendet:
- DAST: Diethylaminoschwefeltrifluorid
- DCC: Dicyclohexylcarbodiimid
- DDQ: Dichlordicyanobenzochinon
- DIBALH: Diisobutylaluminiumhydrid
- KOT: Kalium-tertiär-butanolat
- THF: Tetrahydrofuran
- pTSOH: p-Toluolsulfonsäure
- TMEDA: Tetramethylethylendiamin

### Beispiel 1

Ein Gemisch von 9,2 g 2,6-Difluor-4-2-(p-ethoxyphenyl)-ethyl-phenylboronsäure (hergestellt durch Umsetzung von 3,5-Difluorbenzaldehyd mit p-Ethoxybenzyltriphenylphosphoniumjodid nach Wittig und nachfolgender Hydrierung des erhaltenen Styrolderivates an Pd/C), 6,5 g 1-Brom-4-difluormethylbenzol, 38 ml 2 M wässeriger Na2CO3-Lsg., 0,6 g Tetrakis(triphenylphosphin)palladium-(0) und 75 ml Toluol wird zwei Stunden am Rückfluß gekocht. Die wässerige Phase wird zweimal mit Toluol extrahiert und mit der org. Phase vereinigt. Nach üblicher wässeriger Aufarbeitung und chromatographischer Aufreinigung des Rückstandes erhält man 4-[2-(p-Ethoxyphenyl)-ethyl]-2,6-difluor-4'-difluormethylbiphenyl.

### Beispiele 2 bis 22

Analog erhält man aus den entsprechenden Vorstufen der Formel II die folgenden erfindungsgemäßen Verbindungen:

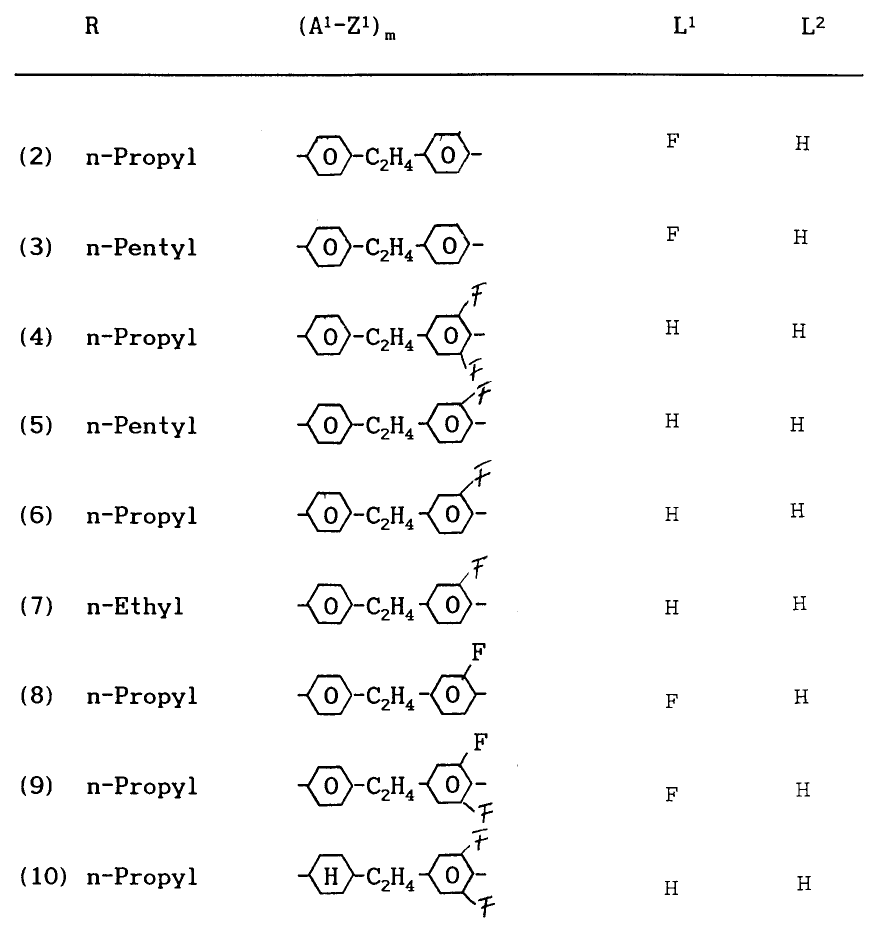

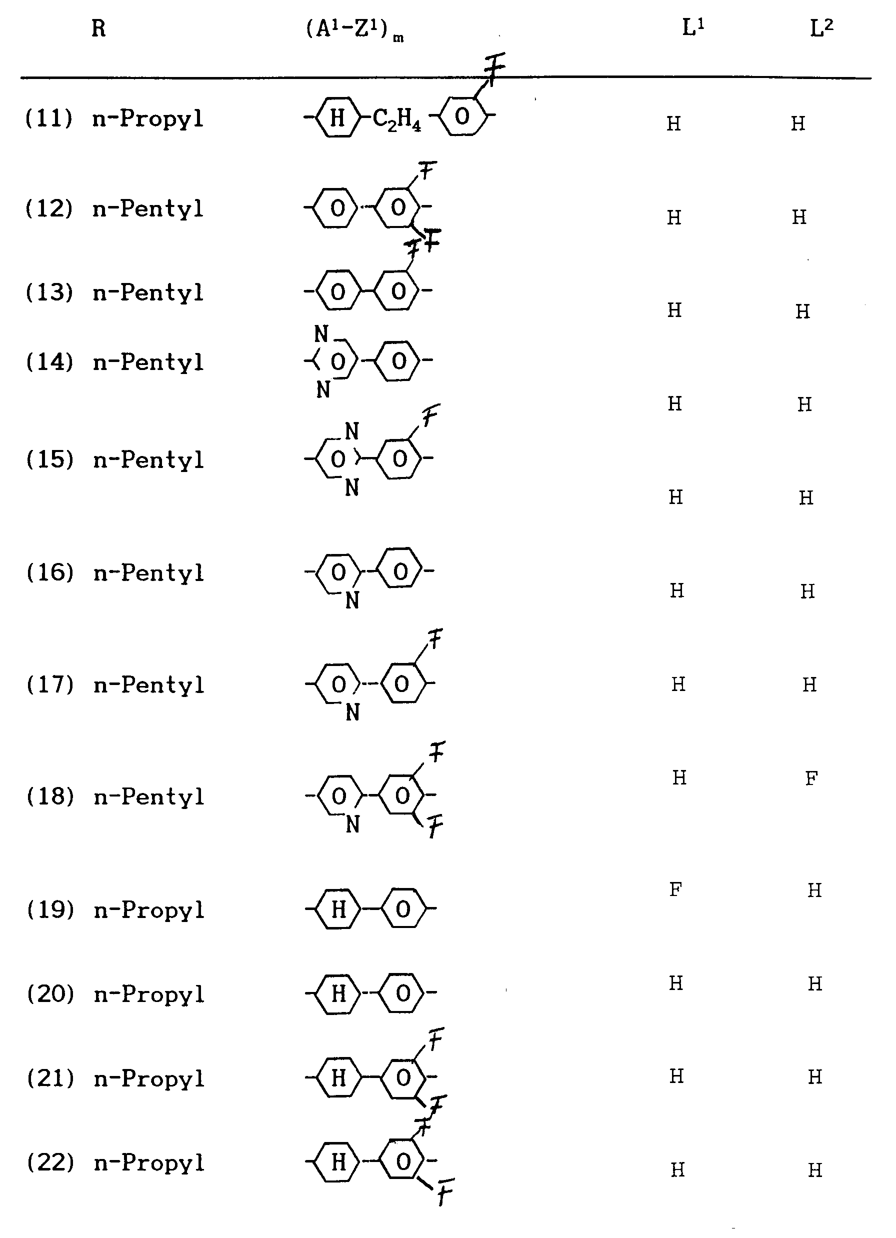

### Beispiel 23

In einen 800-ml-Hastelloy-Autoklaven, der eine Mischung aus 48 g p-(trans-4-n-Propylcyclohexyl)benzaldehyd und 0,1 g Kaliumfluorid enthält, werden unter Kühlung mit einer Aceton-Trockeneis-Mischung 200 g Schwefeltetrafluorid einkondensiert. Anschließend wird der Autoklav 120 h auf 150° erhitzt. Nach Abkühlen auf ca. 30° wird der gasförmige Inhalt des Autoklaven in Waschflaschen mit Natronlauge abgeleitet. Zum Rückstand werden (unter Rühren) nochmals 5 g Kaliumfluorid, danach 50 ml Pentan, dann unter Eiskühlung 10 g Natriumhydrogencarbonat und schließlich 100 ml Wasser zugegeben. Die organische Phase wird abgetrennt, mit wenig wässriger NaHCO₃-Lösung und mit Wasser gewaschen. Nach Abziehen der flüchtigen Bestandteile wird im Hochvakuum (0,1 Torr) bei ca. 180° destilliert. Man erhält p-(trans-4-n-propylcyclohexyl)-difluormethylbenzol, K 14,0 I, 3,7.

Analog werden hergestellt:
p-(trans-4-n-pentylcyclohexyl)-difluormethylbenzol
p-trans-3-(trans-4-ethylcyclohexyl)-cyclohexyl-difluormethylbenzol
p-trans-3-(trans-4-propylcyclohexyl)-cyclohexyl-difluormethylbenzol
p-trans-3-(trans-4-butylcyclohexyl)-cyclohexyl-difluormethylbenzol
p-trans-3-(trans-4-pentylcyclohexyl)-cyclohexyl-difluormethylbenzol,
K 54,0 S_{B} 101,0 N 157,6 I, Δε = +3,2
p-trans-3-(trans-4-heptylcyclohexyl)-cyclohexyl-difluormethylbenzol
p-[trans-3-(trans-4-ethylcyclohexyl)-cyclohexyl]-o-fluordifluormethylbenzol
p-[trans-3-(trans-4-propylcyclohexyl)-cyclohexyl]-o-fluordifluormethylbenzol
p-[trans-3-(trans-4-butylcyclohexyl)-cyclohexyl]-o-fluordifluormethylbenzol
p-[trans-3-(trans-4-pentylcyclohexyl)-cyclohexyl]-o-fluordifluormethylbenzol
4'-(trans-4-ethylcyclohexyl)-4-difluormethylbiphenyl
4'-(trans-4-propylcyclohexyl)-4-difluormethylbiphenyl
4'-(trans-4-butylcyclohexyl)-4-difluormethylbiphenyl
4'-(trans-4-pentylcyclohexyl)-4-difluormethylbiphenyl,
K 122 N 161,8 I, Δε = +4,4
4'-(trans-4-hexylcyclohexyl)-4-difluormethylbiphenyl
4'-(trans-4-heptylcyclohexyl)-4-difluormethylbiphenyl
4'-Ethyl-4'-difluormethylbiphenyl
4'-Propyl-4'-difluormethylbiphenyl
4'-Butyl-4'-difluormethylbiphenyl
4'-Pentyl-4'-difluormethylbiphenyl, K 79 I, Δε = +4,3
4'-Hexyl-4'-difluormethylbiphenyl
4'-Ethoxy-4'-difluormethylbiphenyl
4'-Propoxy-4'-difluormethylbiphenyl
4'-Butoxy-4'-difluormethylbiphenyl
4'-Pentoxy-4'-difluormethylbiphenyl
4'-Hexoxy-4'-difluormethylbiphenyl

### Beispiel 24

### 4-[trans-4-(trans-4-Propylcyclohexyl)-cyclohexyl]-2,6-difluor-difluormethylbenzol

a) 4-[trans-4-(trans-4-Propylcyclohexyl)-cyclohexyl]-2,6-difluorjodbenzol
Zu einer Mischung von 0,094 mol 3-[trans-4-(trans-4-Propylcyclohexyl)-cyclohexyl]-1,5-difluorbenzol, 15 ml TMEDA und 300 ml THF wird bei -65 °C 62 ml einer Lösung von n-Butyllithium (15%ig) in Hexan getropft. Anschließend wird bei -60 °C eine Lösung von 0,094 mol Jod in 50 ml THF zugegeben.
   Nach Zugabe von 60 ml Wasser und 30 ml einer gesättigten Natriumhydrogensulfitlösung wird die wäßrige Phase abgetrennt, die organische Phase wird mit einer gesättigten Kochsalz-Lösung gewaschen, getrocknet über Magnesiumsulfat und im Vakuum eingeengt. Der Rückstand wird aus 200 ml Ethanol umkristallisiert.
b) 4-[trans-4-(trans-4-Propylcyclohexyl)-cyclohexyl]-2,6-difluorbenzaldehyd
   Ein Gemisch aus 0,0224 mol 25a und 50 ml THF wird bei -70 °C mit 14 ml einer 15%igen Lösung von n-Butyllithium in Hexan versetzt. Nach 1stündigem Rühren bei -70 °C wird ein Gemisch aus 0,0224 mol N-Formylpiperidin und 20 ml THF hinzugetropft. Nach Aufwärmen auf -5 °C wird Wasser hinzugegeben.
   Die wäßrige Phase wird abgetrennt, die organische Phase wird mit Wasser und einer gesättigten Bicarbonat-Lösung gewaschen, über Natriumsulfat getrocknet, filtriert und eingeengt. Der Rückstand wird mit Hexan als Laufmittel über eine mit Kieselgel gefüllte Säule chromatographiert, und anschließend aus Hexan umkristallisiert.
c) 4-[trans-4-(trans-4-Propylcyclohexyl)-cyclohexyl]-2,6-difluor-1-difluormethylbenzol
   Zu einem Gemisch aus 0,02 mol Diethylaminoschwefeltrifluorid (DAST) und 5 ml Dichlormethan werden bei Raumtemperatur 0,01 mol 25b gegeben und 2 Stunden zum Sieden erhitzt. Nach zweistündigem Erhitzen werden nochmals 0,01 mol DAST hinzugesetzt und weitere 3 Stunden erhitzt. Nach Abkühlen auf 0 °C werden 25 ml Wasser hinzugesetzt. Nach Abtrennen der wäßrigen Phase wird die organische Phase mit Wasser gewaschen, über Natriumsulfat getrocknet, filtriert und im Vakuum eingeengt. Der Rückstand wird mit Petrolether über eine mit Kieselgel gefüllte Säule chromatograhiert und aus Isopropanol umkristallisiert. Das Produkt zeigt: K 92 N 99,2 I, Δε = +8,99.

### Beispiele 25 bis 37

Analog erhält man aus den entsprechenden Vorstufen die folgenden Verbindungen der Formel Ia

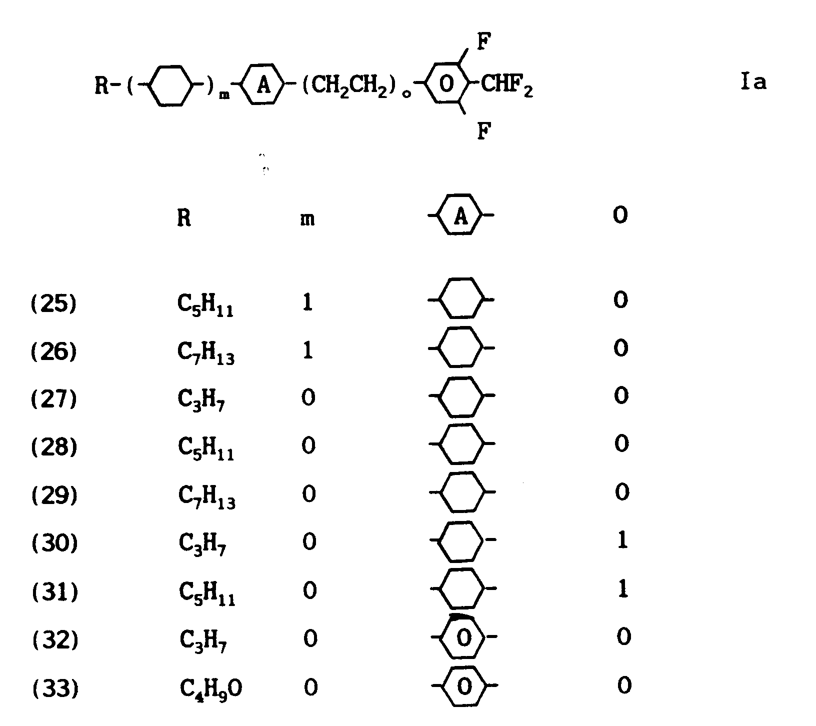

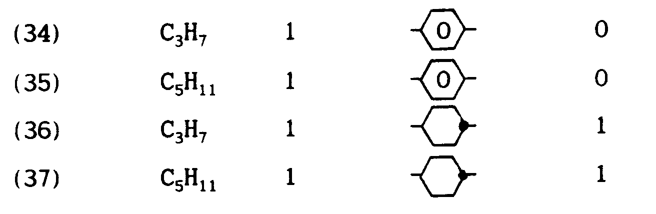

### Beispiel A

Zu einem flüssigkristallinen Medium A bestehend aus
- 22 %: p-(trans-4-Propylcyclohexyl)-ethylbenzol
- 20 %: p-(trans-4-Propylcyclohexyl)-methoxybenzol
- 15 %: p-(trans-4-Propylcyclohexyl)-ethoxybenzol
- 19 %: 4-p-(trans-4-Propylcyclohexyl)-4'-ethylbiphenyl
- 14 %: 4-p-(trans-4-Pentylcyclohexyl)-4'-ethylbiphenyl
- 5 %: 4,4'-Bis-(trans-4-Propylcyclohexyl)-biphenyl
- 5 %: 4-(trans-4-Propylcyclohexyl)-4'-(trans-4-pentylcyclohexyl)-biphenyl
werden jeweils 10 % einer der folgenden erfindungsgemäßen Verbindungen zugegeben:
- A1: p-(trans-4-Propylcyclohexyl)-difluormethylbenzol
- A2: p-[trans-4-(trans-4-Pentylcyclohexyl)-cyclohexyl]-difluormethylbenzol
- A3: 4-(trans-4-Propylcyclohexyl)-4'-difluormethylbiphenyl

Die physikalischen Eigenschaften der so hergestellten flüssigkristallinen Medien können der Tabelle I entnommen werden:

**Tabelle I**

| Medium | Phasenübergang | Δε | Δn | Viskosität m²s⁻¹ (20 °C) |
|---|---|---|---|---|
| A | s < -20 N 72 I | 0,06 | 0,1131 | 11 |
| A1 | S < -20 N 59, SI | 0,43 | 0,1052 | 11,4 |
| A2 | S < -20 N 79,9 I | 0,38 | 0,1122 | 12,7 |
| A3 | S < +10 N 81,6 I | 0,50 | 0,1190 | 13,12 |

### Beispiel B

Man stellt ein flüssigkristallines Medium her bestehend aus
- 9,0 %: p-(trans-4-Pentylcyclohexyl)-fluorbenzol
- 7,2 %: p-(trans-4-Hexylcyclohexyl)-fluorbenzol
- 5,4 %: p-(trans-4-Heptylcyclohexyl)-fluorbenzol
- 7,2 %: p-[trans-4-(trans-4-Ethylcyclohexyl)-cyclohexyl]-trifluormethoxybenzol
- 10,8 %: p-[trans-4-(trans-4-Propylcyclohexyl)-cyclohexyl]-trifluormethoxybenzol
- 8,1 %: p-[trans-4-(trans-4-Butylcyclohexyl)-cyclohexyl]-trifluormethoxybenzol
- 8,1 %: p-[trans-4-(trans-4-Pentylcyclohexyl)-cyclohexyl]-trifluormethoxybenzol
- 10,8 %: 4'-(trans-4-Propylcyclohexyl)-3,4-difluorbiphenyl
- 9,0 %: 4'-(trans-4-Pentylcyclohexyl)-3,4-difluorbiphenyl
- 4,5 %: 2-[trans-4-(trans-4-Propylcyclohexyl)-cyclohexyl]-1-(4-trifluormethoxyphenyl)-ethan
- 4,5 %: 2-[trans-4-(trans-4-Pentylcyclohexyl)-cyclohexyl]-1-(4-trifluormethoxyphenyl)-ethan
- 1,8 %: 4,4'-Bis-(trans-4-Propylcyclohexyl)-2-fluorbiphenyl
- 1,8 %: 4,4'-Bis-(trans-4-Pentylcyclohexyl)-2-fluorbiphenyl
- 1,8 %: 4-(trans-4-Propylcyclohexyl)-4'-(trans-4-pentylcyclohexyl)-2'-fluorbiphenyl
- 10,0 %: 4-[trans-4-(trans-4-Propylcyclohexyl)-cyclohexyl]-2,6-difluor-1-difluormethylbenzol

Dieses Medium weist folgende physikalische Eigenschaften auf:
S < -20 N 89,7 I, Δε 5,58, Δn 0,0954, Viskosität (20 °C): 16,8 mm²s⁻¹

### Beispiel C

Man stellt ein flüssigkristallines Medium her bestehend aus
- 7,0 %: p-(trans-4-Pentylcyclohexyl)-fluorbenzol
- 8,0 %: p-(trans-4-Hexylcyclohexyl)-fluorbenzol
- 6,0 %: p-(trans-4-Heptylcyclohexyl)-fluorbenzol
- 8,0 %: p-(trans-4-Pentylcyclohexyl)-difluormethylbenzol
- 5,0 %: p-[trans-4-(trans-4-Ethylcyclohexyl)-cyclohexyl]-trifluormethoxybenzol
- 6,0 %: p[trans-4-(trans-4-Propylcyclohexyl)-cyclohexyl]-trifluormethoxybenzol
- 6,0 %: p-[trans-4-(trans-4-Butylcyclohexyl)-cyclohexyl]-trifluormethoxybenzol
- 6,0 %: p-[trans-4-(trans-4-Pentylcyclohexyl)-cyclohexyl]-trifluormethoxybenzol
- 9,0 %: p-[trans-4-(trans-4-Propylcyclohexyl)-cyclohexyl]-di fluormethylbenzol
- 9,0 %: p-[trans-4-(trans-4-Pentylcyclohexyl)-cyclohexyl]-difluormethylbenzol
- 8,0 %: 4'-(trans-4-Pentylcyclohexyl)-4-difluormethylbiphenyl
- 8,0 %: 4'-(trans-4-Pentylcyclohexyl)-4-(difluorchlormethyl)-3,5-difluorbiphenyl
- 6,0 %: 4'-(trans-4-Pentylcyclohexyl)-4-difluormethyl-3,5-difluorbiphenyl
- 3,0 %: 4,4'-Bis-(trans-4-pentylcyclohexyl)-2-fluorbiphenyl
- 4,0 %: 4,4'-Bis-(trans-4-propylcyclohexyl)-2-fluorbiphenyl
- 3,0 %: 4-(trans-4-Propylcyclohexyl)-4'-(trans-4-pentylcyclohexyl)-2'-fluorbiphenyl

### Beispiel D

Man stellt ein flüssigkristallines Medium her bestehend aus
- 7,0 %: p-(trans-4-Pentylcyclohexyl)-fluorbenzol
- 6,0 %: p-(trans-4-Hexylcyclohexyl)-fluorbenzol
- 6,0 %: p-(trans-4-Heptylcyclohexyl)-fluorbenzol
- 8,0 %: p-(trans-4-Pentylcyclohexyl)-difluormethylbenzol
- 5,0 %: p-[trans-4-(trans-4-Ethylcyclohexyl)-cyclohexyl]-trifluormethoxybenzol
- 6,0 %: p-[trans-4-(trans-4-Propylcyclohexyl)-cyclohexyl]-trifluormethoxybenzol
- 6,0 %: p-[trans-4-(trans-4-Butylcyclohexyl)-cyclohexyl]-trifluormethoxybenzol
- 6,0 %: p-[trans-4-(trans-4-Pentylcyclohexyl)-cyclohexyl]-trifluormethoxybenzol
- 9,0 %: p-[trans-4-(trans-4-Propylcyclohexyl)-cyclohexyl]-difluormethyl-2,6-difluorbenzol
- 9,0 %: p-[trans-4-(trans-4-Pentylcyclohexyl)-cyclohexyl]-difluormethylbenzol
- 4,0 %: 4'-(trans-4-Pentylcyclohexyl)-3,4-difluorbiphenyl
- 8,0 %: 4'-(trans-4-Propylcyclohexyl)-3,4-difluorbiphenyl
- 5,0 %: 1-[trans-4-(trans-4-Propylcyclohexyl)-cyclohexyl]-2-(trifluormethoxyphenyl)-ethan
- 5,0 %: 1-[trans-4-(trans-4-Propylcyclohexyl)-cyclohexyl]-2-(trifluormethoxyphenyl)-ethan
- 3,0 %: 4,4'-Bis-(trans-4-pentylcyclohexyl)-2-fluorbiphenyl
- 4,0 %: 4,4'-Bis-(trans-4-propylcyclohexyl)-2-fluorbiphenyl
- 3,0 %: 4-(trans-4-Propylcyclohexyl)-4'-(trans-4-pentylcyclohexyl)-2'-fluorbiphenyl

## Patentansprüche (Patentansprüche für folgende(n) Vertragsstaat(en): DE)

1. Difluormethylverbindungen der Formel I, 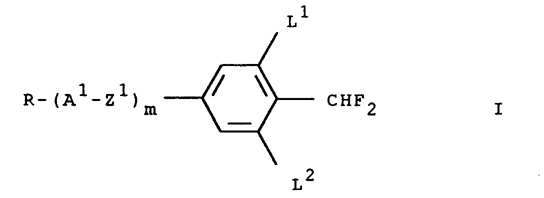 worin
R einen unsubstituierten oder einen mindestens einfach durch Halogen substituierten Alkyl- oder Alkenylrest mit 1 bis 15 C-Atomen, wobei in diesem Rest auch eine oder mehrere CH₂-Gruppen jeweils unabhängig voneinander durch -O-, -S-, 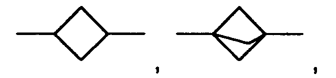 -CO-, -CO-O-, -O-CO- oder -O-CO-O- so ersetzt sein können, daß O-Atome nicht direkt miteinander verknüpft sind,
A¹ einen
(a) trans-1 ,4-Cyclohexylenrest, worin auch eine oder mehrere nicht benachbarte CH₂-Gruppen durch -O- und/oder -S- ersetzt sein können,
(b) 1,4-Phenylenring, worin auch eine oder zwei CH-Gruppen durch N ersetzt sein können,
(c) Rest aus der Gruppe 1,4-Cyclohexenylen, 1,4-Bicyclo[2.2.2]octylen, Piperidin-1,4-diyl, Naphthalin-2,6-diyl, Decahydronaphthalin-2,6-diyl, 1,2,3,4-Tetrahydronaphthalin-2,6-diyl,
wobei die Reste (a) und (b) mit CN oder 1-2 Fluor substituiert sein können,
L¹ und L² jeweils unabhängig voneinander H oder F,
Z¹ -CO-O-, -O-CO-, -CH₂O-, -OCH₂,- -CH₂CH-, -CH=CH-, -C≡C- oder eine Einfachbindung,
m 1, 2 oder 3,
mit der Maßgabe, daß L¹ und/oder L² F bedeuten.

2. Difluormethylverbindungen nach Anspruch 1 der Formel IA 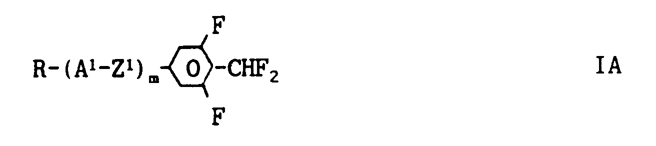 worin R, A¹, Z¹ und m die angegebene Bedeutung besitzen.

3. Difluormethylverbindungen nach Anspruch 2 der Formel IA' 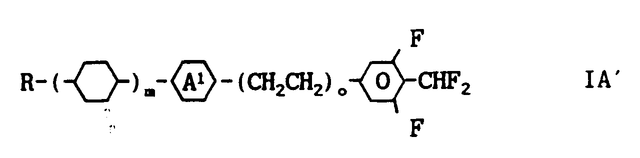 worin R die angegebene Bedeutung besitzt,
m 0 oder 1, 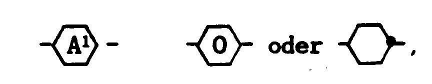 und
o 0 oder 1
bedeuten.

4. Verwendung von Verbindungen der Formel I als Komponenten flüssigkristalliner Medien.

5. Flüssigkristallines Medium mit mindestens zwei flüssigkristallinen Komponenten, dadurch gekennzeichnet, daß es mindestens eine Verbindung der Formel I enthält.

6. Flüssigkristall-Anzeige, dadurch gekennzeichnet, daß es ein flüssigkristallines Medium nach Anspruch 3 enthält.

7. Elektrooptische Anzeige, dadurch gekennzeichnet, daß sie als Dielektrikum ein flüssigkristallines Medium nach Anspruch 3 enthält.

## Patentansprüche (Patentansprüche für folgende(n) Vertragsstaat(en): GB)

1. Difluormethylverbindungen der Formel I, 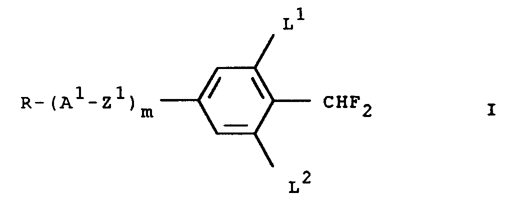 worin
R einen unsubstituierten oder einen mindestens einfach durch Halogen substituierten Alkyl- oder Alkenylrest mit 1 bis 15 C-Atomen, wobei in diesem Rest auch eine oder mehrere CH₂-Gruppen jeweils unabhängig voneinander durch -O-, -S-, 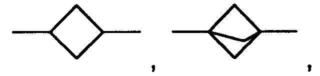 -CO-, -CO-O-, -O-CO- oder -O-CO-O- so ersetzt sein können, daß O-Atome nicht direkt miteinander verknüpft sind,
A¹ einen
(a) trans-1,4-Cyclohexylenrest, worin auch eine oder mehrere nicht benachbarte CH₂-Gruppen durch -O- und/oder -S- ersetzt sein können,
(b) 1,4-Phenylenring, worin auch eine oder zwei CH-Gruppen durch N ersetzt sein können,
(c) Rest aus der Gruppe 1,4-Cyclohexenylen, 1,4-Bicyclo[2.2.2]octylen, Piperidin-1,4-diyl, Naphthalin-2,6-diyl, Decahydronaphthalin-2,6-diyl, 1,2,3,4-Tetrahydronaphthalin-2,6-diyl,
wobei die Reste (a) und (b) mit CN oder 1-2 Fluor substituiert sein können,
L¹ und L² jeweils unabhängig voneinander H oder F,
Z¹ -CO-O-, -O-CO-, -CH₂O-, -OCH₂-, -CH₂CH₂-, -CH=CH-, -C≡C- oder eine Einfachbindung,
m 1, 2 oder 3.

2. Difluormethylverbindungen nach Anspruch 1 der Formel IA 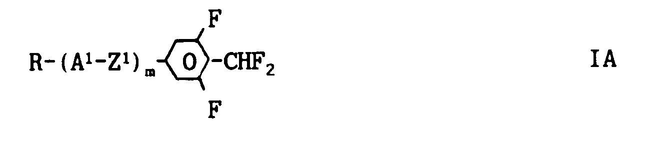 worin R, A¹, Z¹ und m die angegebene Bedeutung besitzen.

3. Difluormethylverbindungen nach Anspruch 2 der Formel IA' 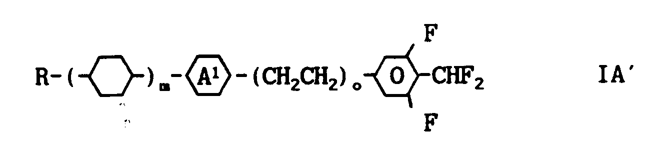 worin R die angegebene Bedeutung besitzt,
m 0 oder 1, 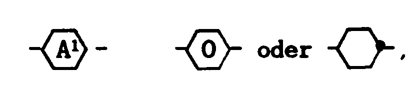 und
o 0 oder 1
bedeuten.

4. Verwendung von Verbindungen der Formel I als Komponenten flüssigkristalliner Medien.

5. Flüssigkristallines Medium mit mindestens zwei flüssigkristallinen Komponenten, dadurch gekennzeichnet, daß es mindestens eine Verbindung der Formel I enthält.

6. Flüssigkristall-Anzeige, dadurch gekennzeichnet daß es ein flüssigkristallines Medium nach Anspruch 3 enthält.

7. Elektrooptische Anzeige, dadurch gekennzeichnet, daß sie als Dielektrikum ein flüssigkristallines Medium nach Anspruch 3 enthält.

## Claims (Claims for the following Contracting State(s): DE)

1. Difluoromethyl compounds of the formula I 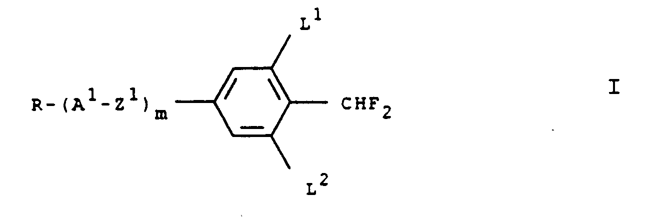 where
R is an alkyl or alkenyl radical having 1 to 15 carbon atoms which is unsubstituted or at least monosubstituted by halogen, it also being possible for one or more CH₂ groups in this radical to be replaced, in each case independently of one another, by -O-, -S-, 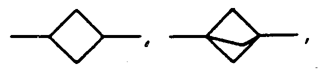 -CO-, -CO-O-, -O-CO- or -O-CO-O- in such a manner that oxygen atoms are not linked directly to one another,
A¹ is
(a) a trans-1,4-cyclohexylene radical in which, in addition, one or more non-adjacent CH₂ groups may be replaced by -O- and/or -S-,
(b) a 1,4-phenylene ring in which, in addition, one or two CH groups may be replaced by N,
(c) a radical from the group comprising 1,4-cyclohexenylene, 1,4-bicyclo[2.2.2]octylene, piperidine-1,4-diyl, naphthalene-2,6-diyl, decahydronaphthalene-2,6-diyl and 1,2,3,4-tetrahydronaphthalene-2,6-diyl,
it being possible for the radicals (a) and (b) to be substituted by CN or monosubstituted or disubstituted by fluorine,
L¹ and L² in each case independently of one another, are H or F,
Z¹ is -CO-O-, -O-CO-, -CH₂O-, -OCH₂-, -CH₂CH₂-, -CH=CH-, -C≡C- or a single bond,
m is 1, 2 or 3,
with the proviso that L¹ and/or L² is F.

2. Difluoromethyl compounds according to Claim 1, of formula IA 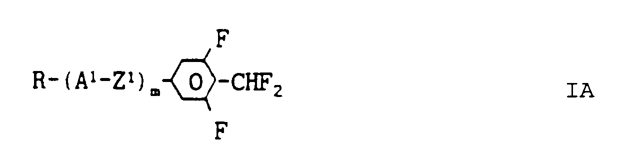 where R, A¹, Z¹ and m are as defined above.

3. Difluoromethyl compounds according to Claim 2, of the formula IA' 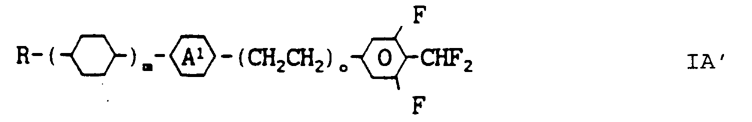 where R is as defined above,
m is 0 or 1, 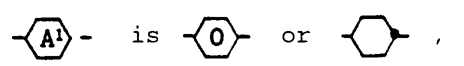 and
o is 0 or 1.

4. Use of compounds of the formula I as components of liquid-crystalline media.

5. Liquid-crystalline medium having at least two liquid-crystalline components, characterised in that it contains at least one compound of the formula I.

6. Liquid-crystal display, characterised in that it contains a liquid-crystalline medium according to Claim 3.

7. Electrooptical display, characterised in that it contains, as dielectric, a liquid-crystalline medium according to Claim 3.

## Claims (Claims for the following Contracting State(s): GB)

1. Difluoromethyl compounds of the formula I 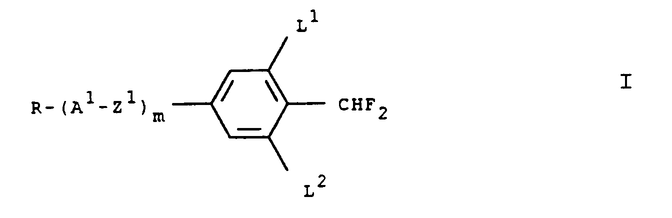 where
R is an alkyl or alkenyl radical having 1 to 15 carbon atoms which is unsubstituted or at least monosubstituted by halogen, it also being possible for one or more CH₂ groups in this radical to be replaced, in each case independently of one another, by -O-, -S-, 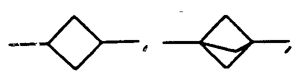 -CO-, -CO-O-, -O-CO- or -O-CO-O- in such a manner that oxygen atoms are not linked directly to one another,
A¹ is
(a) a trans-1,4-cyclohexylene radical in which, in addition, one or more non-adjacent CH₂ groups may be replaced by -O- and/or -S-,
(b) a 1,4-phenylene ring in which, in addition, one or two CH groups may be replaced by N,
(c) a radical from the group comprising 1,4-cyclohexenylene, 1,4-bicyclo[2.2.2]octylene, piperidine-1,4-diyl, naphthalene-2,6-diyl, decahydronaphthalene-2,6-diyl and 1,2,3,4-tetrahydronaphthalene-2,6-diyl,
it being possible for the radicals (a) and (b) to be substituted by CN or monosubstituted or disubstituted by fluorine,
L¹ and L² in each case independently of one another, are H or F,
Z¹ is -CO-O-, -O-CO-, -CH₂O-, -OCH₂-, -CH₂CH₂-, -CH=CH-, -C≡C- or a single bond,
m is 1, 2 or 3,

2. Difluoromethyl compounds according to Claim 1, of formula IA 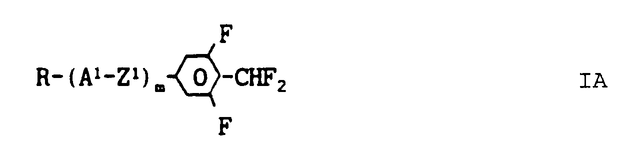 where R, A¹, Z¹ and m are as defined above.

3. Difluoromethyl compounds according to Claim 2, of the formula IA' 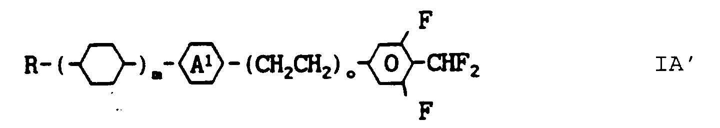 where R is as defined above,
m is 0 or 1, 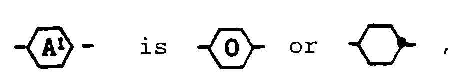 and
o is 0 or 1.

4. Use of compounds of the formula I as components of liquid-crystalline media.

5. Liquid-crystalline medium having at least two liquid-crystalline components, characterised in that it contains at least one compound of the formula I.

6. Liquid-crystal display, characterised in that it contains a liquid-crystalline medium according to Claim 3.

7. Electrooptical display, characterised in that it contains, as dielectric, a liquid-crystalline medium according to Claim 3.

## Revendications (Revendications pour l'(les) Etat(s) contractant(s) suivant(s): DE)

1. Composés difluorométhylés de formule I 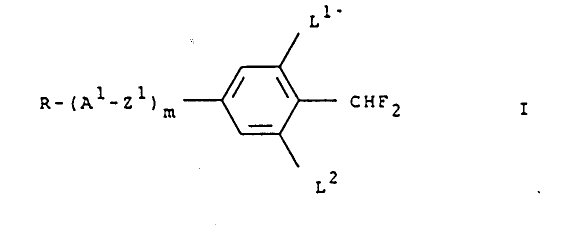 dans laquelle
R est un radical alkyle ou alcényle ayant de 1 à 15 atomes de carbone, non-substitué ou substitué au moins une fois par un atome d'halogène, où, dans ce radical, un ou plusieurs groupes CH₂ peuvent aussi, chacun indépendamment des autres, être remplacés par -O-, -S-, 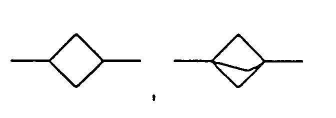 -CO-, -CO-O-, -O-CO- ou -O-CO-O-, de façon que des atomes d'oxygène ne soient pas directement reliés l'un à l'autre,
A¹ est :
(a) un radical trans-1,4-cyclohexyle, où un ou plusieurs groupes CH₂ non-voisins peuvent aussi être remplacés par -O- et/ou -S-,
(b)un noyau 1,4-phénylène, où un ou deux groupes CH peuvent aussi être remplacés par N,
(c) un radical choisi dans le groupe comprenant le 1,4-cyclohexénylène, le 1,4-bicyclo[2.2.2]-octylène, pipéridine-1,4-diyle, naphtalène-2,6-diyle, décahydronaphtalène-2,6-diyle, 1,2,3,4-tétrahydronaphtalène-2,6-diyle,
où les radicaux (a) et (b) peuvent être substitués par CN ou par 1 ou 2 atomes de fluor,
L¹ et L² représentent chacun indépendamment de l'autre H ou F,
Z¹ est -CO-O-, -O-CO-, -CH₂O-, -OCH₂-, -CH₂CH₂-, -CH=CH-, -C(C- ou une liaison simple,
m vaut 1, 2 ou 3,
à la condition que L¹ et/ou L² représentent F.

2. Composés difluorométhylés selon la revendication 1 de formule IA 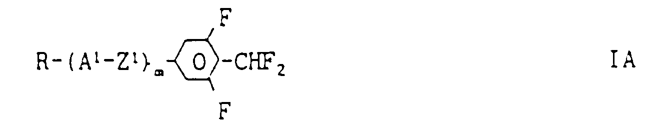 dans laquelle R, A¹, Z¹ et m ont les significations indiquées.

3. Composés difluorométhylés selon la revendication 2 de formule IA' 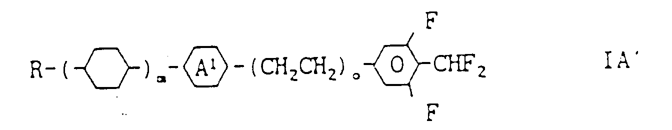 dans laquelle R a les significations données ci-dessus,
m vaut 0 ou 1 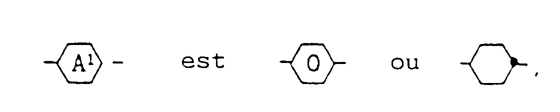 et
o vaut 0 ou 1.

4. Utilisation de composés de formule I en tant que constituants de milieux à cristaux liquides.

5. Milieu à cristaux liquides comportant au moins deux constituants de type cristal liquide, caracterisé en ce qu'il contient au moins un composé de formule I.

6. Affichage à cristaux liquides, caractérisé en ce qu'il contient un milieu à cristaux liquides selon la revendication 3.

7. Affichage électro-optique, caractérisé en ce qu'il contient en tant que diélectrique un milieu à cristaux liquides selon la revendication 3.

## Revendications (Revendications pour l'(les) Etat(s) contractant(s) suivant(s): GB)

1. Composés difluorométhylés de formule I 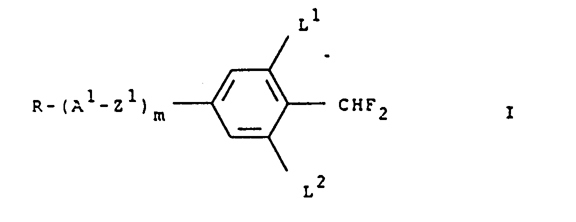 dans laquelle
R est un radical alkyle ou alcényle ayant de 1 à 15 atomes de carbone, non-substitué ou substitué au moins une fois par un atome d'halogène, où, dans ce radical, un ou plusieurs groupes CH₂ peuvent aussi, chacun indépendamment des autres, être remplacés par -O-, -S-, 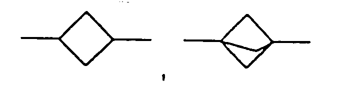 -CO-, -CO-O-, -O-CO- ou -O-CO-O-, de façon que des atomes d'oxygène ne soient pas directement reliés l'un à l'autre,
A¹ est :
(a) un radical trans-1,4-cyclohexyle, où un ou plusieurs groupes CH₂ non-voisins peuvent aussi être remplacés par -O- et/ou -S-,
(b) un noyau 1,4-phénylène, où un ou deux groupes CH peuvent aussi être remplacés par N,
(c) un radical choisi dans le groupe comprenant le 1,4-cyclohexénylène, le 1,4-bicyclo[2.2.2]-octylène, pipéridine-1,4-diyle, naphtalène-2,6-diyle, décahydronaphtalène-2,6-diyle, 1,2,3,4-tétrahydronaphtalène-2,6-diyle,
où les radicaux (a) et (b) peuvent être substitués par CN ou par 1 ou 2 atomes de fluor,
L¹ et L² représentent chacun indépendamment de l'autre H ou F,
Z¹ est -CO-O-, -O-CO-, -CH₂O-, -OCH₂-, -CH₂CH₂-, -CH=CH-, -C(C- ou une liaison simple,
m vaut 1, 2 ou 3.

2. Composés difluorométhylés selon la revendication 1 de formule IA 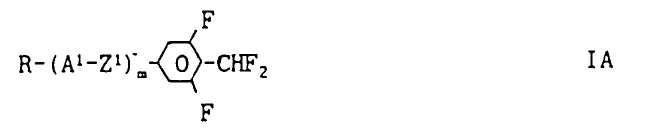 dans laquelle R, A¹, Z¹ et m ont les significations indiquées.

3. Composés difluorométhylés selon la revendication 2 de formule IA' 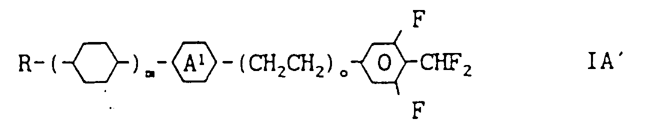 dans laquelle R a les significations données ci-dessus,
m vaut 0 ou 1 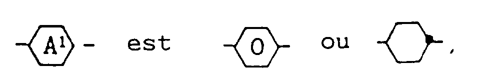 et
o vaut 0 ou 1.

4. Utilisation de composés de formule I en tant que constituants de milieux à cristaux liquides.

5. Milieu à cristaux liquides comportant au moins deux constituants de type cristal liquide, caractérisé en ce qu'il contient au moins un composé de formule I.

6. Affichage à cristaux liquides, caractérisé en ce qu'il contient un milieu à cristaux liquides selon la revendication 3.

7. Affichage électro-optique, caractérisé en ce qu'il contient en tant que diélectrique un milieu à cristaux liquides selon la revendication 3.
